# EUROPEAN PATENT APPLICATION

(11) **EP 3 485 965 A1**
(43) Date of publication of application: **22.05.2019**
(21) Application number: 17827586.3
(22) Date of filing: 10.07.2017
(51) Int. Cl.: B01D 69/00, B01D 71/34, B01D 71/36, C07C 229/12, C07C 251/24, C07F 9/54, C07F 15/06

(54) **OXYGEN SEPARATION MEMBRANE**

(30) Priority: 15.07.2016 JP 2016140446
(71) Applicant: Sharp Kabushiki Kaisha, Sakai City, Osaka 590-8522 (JP); National University Corporation Kobe University, Kobe-shi, Hyogo 657-8501 (JP)
(72) Inventor: NAKANISHI, Yasunori, Sakai City Osaka 590-8522 (JP); MATSUYAMA, Hideto, Kobe-shi Hyogo 657-8501 (JP); KAMIO, Eiji, Kobe-shi Hyogo 657-8501 (JP); MATSUOKA, Atsushi, Kobe-shi Hyogo 657-8501 (JP)
(74) Representative: Müller-Boré & Partner Patentanwälte PartG mbB
(86) International application number: PCT/JP2017/025137
(87) International publication number: WO 2018/012459

(57) **Abstract**

An oxygen separation membrane includes a porous material and a liquid complex contained in the porous material. The complex contains a metal salen complex or a derivative thereof and a first ionic liquid. The first ionic liquid is constituted by an anion having an amine structure and an imidazolium cation, an aliphatic quaternary phosphonium cation, or an ammonium cation that have alkyl chains, alkylene oxide chains, or alkyl ether chains with each 2 to 20 carbon atoms. The anion of the first ionic liquid is axially coordinated to a central metal ion of the metal salen complex or the derivative thereof.

## Description

### Technical Field

The present invention relates to an oxygen separation membrane containing, as a constituent component, a molecule that selectively reacts with oxygen to efficiently separate oxygen from a mixed gas containing oxygen.

### Background Art

A gas separation technique/process is promising for not only industrial use but also general use because such a technique/process can be used for, for example, purification of exhaust gas, deodorizing treatment, extraction of components, and energy saving.

An oxygen gas is one of the most familiar gases in our lives, and it is not an exaggeration to say that oxygen is involved in all chemical reactions such as respiration, combustion, catalysis, and molecular synthesis. If operations such as efficient absorption of oxygen, concentration of oxygen, and storage of oxygen can be controlled with a simple system, such an operation is expected to be useful in many fields ranging from household to industry. However, the controlled handling of oxygen is challenging.

At present, high-concentration oxygen is generated by a cryogenic separation method or an adsorptive separation method in systems of facilities that produce a high-pressure oxygen gas and oxygen enrichers. However, since these methods require temperature or pressure control, a high-energy process needs to be employed or the system size is large. As a result, such a method is employed only in specialty uses.

In contrast, a membrane separation method does not require temperature or pressure control with high energy consumption. Therefore, a compact system can be realized with low energy, and the membrane separation method is a convenient method suitable for oxygen supply.

A gas separation membrane capable of performing selective permeation of particular gas molecules is important to achieve efficient membrane separation. The gas separation membrane allows particular gas molecules to permeate more easily than other gas molecules by using the molecular size and the difference in reactivity, and a mixed gas containing target molecules such as an organic gas, an oxide gas, carbon dioxide, and water vapor can be separated into each components in accordance with the purpose. The most generally used gas separation membrane is made of a polymer material and is used as a useful separation membrane with features such as the pore diameter of the porous membrane, the lyophilic and lyophobic properties, and the properties of an acid and a base. However, in particular when oxygen and nitrogen in the air are separated from each other, it is extremely difficult to perform oxygen separation using the pore shape because their molecular sizes are substantially the same. Thus, high selectivity is not achieved when a polymer membrane is used.

To achieve highly efficient oxygen separation with a membrane, the application of a metal complex that has selective reactivity with oxygen is known to be effective. A typical example of an adsorption-desorption reaction of oxygen is an oxygen transport reaction of a heme protein such as hemoglobin that functions in a living body, which is caused by adsorption and desorption of oxygen to a porphyrin-iron ion complex. Japanese Unexamined Patent Application Publication No. 2007-209543 (PTL 1) discloses that a polyethylene glycol-modified serum albumin-metal porphyrin composite exhibits reversible adsorption and desorption of oxygen as a biomimetic model and proposes that the composite is applied to an oxygen adsorbing membrane. The oxygen adsorbing membrane is formed from water or an ethanol solution, and therefore may be subjected to elution to water or the organic solvent, which poses a problem in terms of stability as a permeable membrane. Furthermore, in consideration of living body-derived raw materials, complicated synthesis process, and stability of functional molecular structure, it is difficult to reproduce the functional structure and practically use the oxygen adsorbing membrane.

Japanese Unexamined Patent Application Publication No. 2014-114366 (PTL 2) proposes a polymer compound obtained by adding a metal porphyrin complex to a polymer framework and an oxygen permeable membrane containing the polymer compound. The porphyrin complex-containing polymer compound herein has a large ratio of complex structures relative to polymer structures, and thus has a higher oxygen permeability than known mixture membranes of polymers and complexes and also exhibits a high oxygen selective permeability even at an oxygen partial pressure in the air. However, since the diffusion of oxygen in the membrane is suppressed by immobilizing such a large complex structure to a polymer framework, the oxygen permeable membrane has a lower permeability than separation membranes for other gases.

Another useful complex having a metal complex structure that has reactivity with oxygen is a metal salen complex. Salen ("N,N'-bis(salicylidene)ethylenediamine" or "N,N'-bis(2-hydroxybenzylidene)ethylenediamine") has a planar structure as a tetradentate ligand of a metal ion, and another ligand and an oxygen molecule can be axially coordinated to the metal ion. Thus, salen has a function of oxygen absorption.

Japanese Unexamined Patent Application Publication No. 6-340683 (PTL 3) and Japanese Unexamined Patent Application Publication No. 9-151192 (PTL 4) propose various derivatives of salen complexes. Salen complexes are generally solid and thus need to be processed into a membrane or the like when applied to oxygen separation. Therefore, improvements in the solubility of complex molecules and the stability of a structure have been studied in addition to the oxygen adsorbability/desorbability of the complex.

Lloyd M. Robeson "The upper bound revisited", Journal of Membrane Science, 2008, Vol. 320, pp. 390-400 (NPL 1) summarizes the relationship between the permeability and the permeation selectivity of oxygen and nitrogen of known oxygen separation membranes. However, known oxygen separation membranes have insufficient performance and a further improvement in performance has been desired.

### Citation List

### Patent Literature

PTL 1: Japanese Unexamined Patent Application Publication No. 2007-209543
PTL 2: Japanese Unexamined Patent Application Publication No. 2014-114366
PTL 3: Japanese Unexamined Patent Application Publication No. 6-340683
PTL 4: Japanese Unexamined Patent Application Publication No. 9-151192

### Non Patent Literature

NPL 1: Lloyd M. Robeson "The upper bound revisited", Journal of Membrane Science, 2008, Vol. 320, pp. 390-400

### Summary of Invention

### Technical Problem

To achieve a high selective permeability of an oxygen separation membrane, a metal complex that selectively reacts with oxygen molecules needs to be accumulated in a membrane with high density and additionally to be improved the diffusibility of the oxygen molecules through reaction sites. However, a solid metal complex needs to be dissolved in an organic solvent once for membrane formation, which makes it difficult to achieve the high density in consideration of the limited solubility of the complex. Furthermore, if complex molecules are immobilized on the membrane structure itself, the reaction sites with oxygen are restricted, which suppresses the movement of oxygen in the membrane and decreases the permeability. That is, it is believed that the performance of the complex molecules cannot be sufficiently exhibited when the complex molecules are used in the form of a membrane, and therefore such a complex has not been put into practical use.

Accordingly, it is an object of the present invention to provide an oxygen separation membrane that exhibits a high oxygen selective permeability. In the oxygen separation membrane, a metal complex that selectively reacts with oxygen is stably present in pores of a porous material and functionally acts as an oxygen carrier.

### Solution to Problem

As a result of studies on a membrane structure to achieve the above object, the present inventors have found the following and have completed the present invention. That is, the present inventors have contrived a molecular structure in which a salen complex itself is liquefied by coordinating a nonvolatile ionic liquid as an axial ligand of the salen complex, and an impregnated membrane is formed by impregnating a porous membrane with the salen complex together with an auxiliary ligand that stabilizes the function of the complex. Consequently, a salen complex having a function as an oxygen carrier can be accumulated with high density to provide a membrane having a high oxygen permeability.

The present invention provides an oxygen separation membrane including a porous material and a liquid complex contained in the porous material
wherein the complex contains a metal salen complex or a derivative thereof and a first ionic liquid,
the first ionic liquid is constituted by an anion having an amine structure and an imidazolium cation, an aliphatic quaternary phosphonium cation, or an ammonium cation that have alkyl chains, alkylene oxide chains, or an alkyl ether chains with each 2 to 20 carbon atoms, and
the anion of the first ionic liquid is axially coordinated to a central metal ion of the metal salen complex or the derivative thereof.

### Advantageous Effects of Invention

According to the present invention, an oxygen separation membrane that exhibits a high oxygen selective permeability can be provided. In the oxygen separation membrane, a metal complex that selectively reacts with oxygen is stably present in pores of a porous material and functionally acts as an oxygen carrier.

The oxygen separation membrane according to the present invention has the following preferred embodiments.
(1) The first ionic liquid contains a secondary amine anion.
(2) The first ionic liquid contains an N-methylglycine anion.
(3) The complex further contains a second ionic liquid having compatibility with the first ionic liquid, and the anion of the first ionic liquid is coordinated as one of axial ligands of the central metal ion of the metal salen complex or the derivative thereof.
(4) The second ionic liquid contains a bis(trifluoromethanesulfonyl)imide anion.
(5) The first ionic liquid and/or the second ionic liquid contains any one of a trihexyl(tetradecyl)phosphonium cation, a tributyl-n-octylphosphonium cation, and a triethylpentylphosphonium cation.
(6) The metal salen complex is N,N'-bis(salicylidene)ethylenediaminocobalt(II).
(7) The porous material is a hydrophilic ethylene tetrafluoride resin or a hydrophilic polyvinylidene fluoride resin.
(8) The porous material has a membrane shape with a pore diameter of 0.1 to 0.5 µm.
(9) The oxygen separation membrane has an oxygen permeability of 10 Barrer (1 Barrer = 1 × 10⁻¹⁰ cm³(STP)·cm/(cm²·s·cmHg)) or more at an oxygen partial pressure in air, and a ratio of a permeation separation factor of oxygen to a permeation separation factor of nitrogen is 2 or more.

### Brief Description of Drawings

Fig. 1 schematically illustrates a gas permeation tester configured to measure a gas permeation amount by a sweep method.
Fig. 2 illustrates the relationship between the oxygen partial pressure and the oxygen permeability of oxygen separation membranes in Examples 1 and 2.
Fig. 3 illustrates the relationship between the oxygen partial pressure and the oxygen permeability of oxygen separation membranes in Examples 3 and 4.
Fig. 4 illustrates the relationship between the oxygen partial pressure and the oxygen permeability of oxygen separation membranes in Examples 5 and 6.
Fig. 5 illustrates the oxygen permeability and the permeation selectivity of oxygen and nitrogen of oxygen separation membranes in Examples 1 to 6 and the previously reported paper.

### Description of Embodiments

Hereafter, an embodiment of the present invention will be described in detail, but the present invention is not limited thereto.

An oxygen separation membrane according to the present invention is an oxygen separation membrane including a porous material and a liquid complex contained in the porous material,
wherein the complex contains a metal salen complex or a derivative thereof and a first ionic liquid,
the first ionic liquid is constituted by an anion having an amine structure and an imidazolium cation, an aliphatic quaternary phosphonium cation, or an ammonium cation that have alkyl chains, alkylene oxide chains, or an alkyl ether chains with each 2 to 20 carbon atoms, and
the anion of the first ionic liquid is axially coordinated to a central metal ion of the metal salen complex or the derivative thereof.

In the present invention, the "derivative of a metal salen complex" refers to a metal salen complex in which a hydrogen atom on a carbon atom of a salen structure is replaced with another substituent.

The oxygen separation membrane according to the present invention is mainly constituted by a porous material that forms a framework to ensure the membrane strength, a liquid complex having selective gas permeability in the porous material, and an ionic liquid that stably surrounds the complex. An anion containing a basic nitrogen atom is coordinated as one axial ligand of a central metal ion of the metal salen complex in a planar coordination, and the anion constitutes an ionic liquid with a countercation of the anion. The affinity of an oxygen molecule that is coordinated as the other axial ligand of the central metal ion advantageously increases as the basicity of nitrogen on the anion increases. Therefore, the anion used in the present invention preferably has a secondary amine structure.

Herein, the coordination of an anion of an ionic liquid to a metal ion of a metal salen complex can be confirmed by, for example, visible and ultraviolet spectrophotometry or observation of a coloration state as described in Examples.

### (Metal salen complex)

The metal salen complex that can be used in the present invention is a publicly known metal complex having a structure in which salen or a salen derivative having a substituent is coordinated to a metal ion as a tetradentate ligand. The metal salen complex is represented by, for example, general formula (Chem. 1). 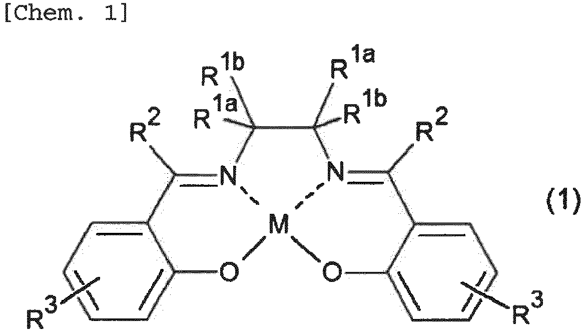 (In the formula, M represents a metal ion and R^{1a}, R^{1b}, R², and R³ are the same or different, each representing a hydrogen atom, a halogen atom, an alkyl group or a haloalkyl group having 1 to 6 carbon atoms, an alkoxy group having 1 to 6 carbon atoms, an acyl group having 2 to 6 carbon atoms, an amino group, a nitro group, a nitrile group (cyano group), a vinyl group, or an aryl group or a heteroaryl group having 6 to 12 carbon atoms, where R^{1a} and R^{1b} may bond to each other through an atom or an atomic group thereof to form a ring structure.)

Various metal ions of, for example, cobalt, iron, manganese, nickel, and copper can be subjected to coordination with a molecule of a metal salen or a derivative thereof. A cobalt(II) ion is most preferably used in terms of adsorption of oxygen molecules.

The substituents R^{1a}, R^{1b}, R², and R³ in the general formula (1) will be described.

Examples of the halogen atom include fluorine, chlorine, bromine, and iodine.

Examples of the alkyl group having 1 to 6 carbon atoms include linear or branched alkyl groups such as methyl, ethyl, n-propyl, isopropyl, n-butyl, isobutyl, sec-butyl, tert-butyl, n-pentyl, neopentyl, and n-hexyl.

Examples of the haloalkyl group having 1 to 6 carbon atoms include alkyl groups obtained by substituting any hydrogen atom on the above alkyl group with the above halogen atom, such as fluoromethyl, chloromethyl, bromomethyl, and trifluoromethyl.

Examples of the alkoxy group having 1 to 6 carbon atoms include linear or branched alkoxy groups such as methoxy, ethoxy, n-propoxy, isopropoxy, n-butoxy, n-pentoxy, and n-hexoxy.

Examples of the acyl group having 2 to 6 carbon atoms include aliphatic acyl groups such as formyl, acetyl, propionyl, butyryl, isobutyryl, valeryl, isovaleryl, pivaloyl, and hexanoyl.

Examples of the aryl group and heteroaryl group having 6 to 12 carbon atoms include phenyl, tolyl, xylyl, fluorophenyl, chlorophenyl, bromophenyl, and naphthyl.

R^{1a} and R^{1b} may be the same or different and may bond to each other through an atom or an atomic group thereof to form a ring structure. The substituent R³ may have any substitution position.

The metal salen complex can be produced as follows. Salen or a salen derivative is synthesized by causing a dehydration condensation reaction of salicylaldehyde and ethylenediamine in a solvent such as ethanol. Then, the obtained salen or salen derivative serving as a ligand is reacted with a metal ion under basic conditions. Alternatively, the metal salen complex can be produced by simultaneously adding a metal acetate during the synthesis of salen or a salen derivative.

Salen derivatives having various structures can be produced by using substitution products of salicylaldehyde and ethylenediamine serving as raw materials for synthesis.

Examples of the substitution product of salicylaldehyde include dihydroxybenzaldehyde, chlorosalicylaldehyde, bromosalicylaldehyde, fluorosalicylaldehyde, aminosalicylaldehyde, methylsalicylaldehyde, tert-butylsalicylaldehyde, methoxysalicylaldehyde, and ethoxysalicylaldehyde.

Examples of the substitution product of ethylenediamine include 1,2-dimethylethylenediamine, 1,1,2,2-tetramethylethylenediamine, 1,2-cyclohexanediamine, and 1,2-diphenylethylenediamine.

When cobalt, which is a metal most suitable for adsorption of oxygen molecules, is used, examples of the metal salen derivative obtained by combining the above materials include cobalt salens, that is, N,N'-bis(salicylidene)ethylenediaminocobalt(II), N,N'-bis(salicylidene)-1,2-dimethylethylenediaminocobalt(II), N,N'-bis(salicylidene)-1,1,2,2-tetramethylethylenediaminocobalt(II), N,N'-bis(salicylidene)-1,2-cyclohexaneethylenediaminocobalt(II), N,N'-bis(3-methylsalicylidene)ethylenediaminocobalt(II), N,N'-bis(5-methylsalicylidene)ethylenediaminocobalt(II), N,N'-bis(3-ethoxysalicylidene)ethylenediaminocobalt(II), N,N'-bis(3,5-di-tert-butylsalicylidene)-1,2-cyclohexanediaminocobalt(II), and N,N'-bis(3-ethoxysalicylidene)-1,1,2,2-tetramethylethylenediaminocobalt(II). Among them, N,N'-bis(salicylidene)ethylenediaminocobalt(II) is particularly preferably used in terms of oxygen absorption.

Furthermore, cobalt salen complexes described or cited in Japanese Unexamined Patent Application Publication No. 6-340683, Pier Giorgio Cozzi, "Metal-Salen Schiff base complexes in catalysis: practical aspects", Chemical Society Reviews, 2004, Vol. 33, pp. 410-421, and Eric C. Niederhoffer and two others, "Thermodynamics of Oxygen Binding in Natural and Synthetic Dioxygen Complexes", Chemical Reviews, 1984, Vol. 84, pp. 137-203 can be used.

### (Ionic liquid)

The oxygen separation membrane according to the present invention contains an ionic liquid. One ionic liquid is a first ionic liquid that is an essential component. The first ionic liquid is axially coordinated to a central metal ion so as to contribute to an increase in the affinity for oxygen of the metal salen complex. The other ionic liquid is a second ionic liquid that is an optional component. The second ionic liquid is an ionic liquid that has compatibility (soluble with each other) with the first ionic liquid. The second ionic liquid facilitates the coordination of an oxygen molecule to a central metal ion in the axial direction, the movement between coordination sites, and the diffusion inside the membrane and furthermore contributes to the stabilization of a structure of the metal salen complex in the membrane.

### (First ionic liquid)

As described above, the first ionic liquid preferably contains an anion having a secondary amine structure and particularly preferably contains an N-alkylamino acid that is generally used as an anion of an ionic liquid and has an alkyl chain provided on an amino group of an amino acid so as to have a secondary amine structure. The number of carbon atoms of the alkyl chain may be any of 1 to 8. However, the influence of steric hindrance during coordination increases as the number of carbon atoms increases. Therefore, the number of carbon atoms is preferably small and the alkyl chain is preferably methyl and ethyl and particularly preferably methyl. For the selection of the type of amino acid, glycine, which has the smallest molecular weight, is preferred in consideration of steric factor during coordination. Thus, the anionic ligand is particularly preferably N-methylglycine (aminoacetic acid).

### (Second ionic liquid)

The second ionic liquid plays an important role in improving the oxygen permeability of the membrane. The second ionic liquid also has an appropriate coordination strength for the metal ion of the metal salen complex as in the case of the first ionic liquid. When oxygen molecules are present near the metal ion, the anion of the first ionic liquid is coordinated as one axial ligand and an oxygen molecule is coordinated as the other axial ligand. If oxygen molecules are not present near the metal ion because of elimination of oxygen molecules, it is believed that the anion of the first ionic liquid is coordinated as one axial ligand and the anion of the second ionic liquid is coordinated as the other axial ligand. If the anion of the second ionic liquid is not present, the anion of the first ionic liquid is coordinated as both axial ligands and the metal salen complex is precipitated as a solid. The state returns to the original state again when oxygen is provided, but the presence of the second ionic liquid is important to keep a stable liquid state.

The complex preferably further contains the second ionic liquid having compatibility with the first ionic liquid, and the anion of the first ionic liquid is preferably coordinated as one of axial ligands of a central metal ion of the metal salen complex or the derivative thereof.

In other words, the oxygen separation membrane according to the present invention is preferably an oxygen separation membrane including a porous material; a metal salen complex or a derivative thereof; a first ionic liquid constituted by an anion having an amine structure and an imidazolium cation, an aliphatic quaternary phosphonium cation, or an ammonium cation that have alkyl chains, alkylene oxide chains, or an alkyl ether chains with each 2 to 20 carbon atoms; and a second ionic liquid having compatibility with the first ionic liquid, wherein a liquid complex in which the anion of the first ionic liquid is coordinated as one of axial ligands of a central metal ion of the metal salen complex or the derivative thereof is contained in the porous material.

The complex of the metal salen complex and the first ionic liquid is often a high viscosity liquid because of its large molecular size. Therefore, a low-viscosity ionic liquid having good compatibility with the first ionic liquid can be added to increase the mobility of the complex, thereby improving the diffusibility of oxygen that permeates through the membrane. Furthermore, the ionic liquid has no volatility and thus has higher stability in the membrane than typical organic solvents.

Thus, the second ionic liquid added to the oxygen separation membrane according to the present invention desirably has a moderate coordinate interaction for the complex and a low viscosity. In consideration of the foregoing, the anion of the second ionic liquid is preferably a bis(trifluoromethanesulfonyl)imide anion among anions of typical ionic liquids because the bis(trifluoromethanesulfonyl)imide anion has a coordinating amino group and achieves low viscosity in the form of an ionic liquid.

Countercations for the anions of the first ionic liquid and the second ionic liquid may be the same or different. The substance that can be used as a cation of the first ionic liquid is, for example, a phosphonium, an ammonium, or an imidazolium that constitutes a typical ionic liquid and is preferably an imidazolium, an aliphatic quaternary phosphonium, or an ammonium that have alkyl chains, alkylene oxide chains, or alkyl ether chains with each 2 to 20 carbon atoms.

The cation of the second ionic liquiccd can be widely selected from, for example, the same cations of the first ionic liquid and pyridinium, pyrrolidinium, and sulfonium used as cations of typical ionic liquids as long as the compatibility is suitable.

The ionic liquid can be prepared by an anion exchange reaction of the above compound to serve as an anion and an imidazolium salt, a phosphonium salt, or an ammonium salt to serve as a cation.

Examples of the imidazolium salt include 1,3-dimethylimidazolium bromide, 1-ethyl-3-methylimidazolium bromide, 1-methyl-3-propylimidazolium bromide, 1-butyl-3-methylimidazolium bromide, 1-hexyl-3-methylimidazolium bromide, 1-decyl-3-methylimidazolium bromide, 1-methyl-3-octylimidazolium bromide, 1-ethyl-2,3-dimethylimidazolium bromide, 1-butyl-2,3-dimethylimidazolium bromide, 1-hexyl-2,3-dimethylimidazolium bromide, 1-butyl-3-ethylimidazolium bromide, 1,3-dibutylimidazolium bromide, 1-butyl-3-hexylimidazolium bromide, 1-butyl-3-octylimidazolium bromide, 1-methoxyethyl-3-methylimidazolium bromide, 1-methoxybutyl-3-methylimidazolium bromide, and chlorides corresponding to these bromides.

Examples of the phosphonium salt and the ammonium salt include tetramethylphosphonium bromide, tetraethylphosphonium bromide, tetrabutylphosphonium bromide, tetrahexylphosphonium bromide, triethylhexylphosphonium bromide, triethyloctylphosphonium bromide, triethyl(2-methoxyethyl)phosphonium bromide, tributyloctylphosphonium bromide, tributyldodecylphosphonium bromide, tributyl(2-methoxyethyl)phosphonium bromide, trihexyldodecylphosphonium bromide, trihexyl(tetradecyl)phosphonium bromide, and chlorides corresponding to these bromides.

The combinations of the imidazolium salt, the phosphonium salt, or the ammonium salt and the anion coordinated as an axial ligand of the metal salen complex do not always have a liquid state at room temperature. Therefore, triethyloctylphosphonium bromide, tributyloctylphosphonium bromide, trihexyl(tetradecyl)phosphonium bromide, and triethylpentylphosphonium bromide are preferred because such a substance has a low melting point and thus tends to be present in the form of an ionic liquid when the cation is combined with each anion. In particular, trihexyl(tetradecyl)phosphonium bromide, tributyl-n-octylphosphonium bromide, and triethylpentylphosphonium bromide are preferred because ionic liquids are formed by combination with many anions.

### (Porous material)

The porous material that can be used in the present invention may be any porous material as long as the porous material stably holds the liquid complex containing the ionic liquid in the pores thereof and has such a strength that the porous material can stand on its own as a gas permeable membrane. A hydrophilic material is preferred from the viewpoint of high affinity for the complex and ease of containment in pores. Furthermore, a hydrophilic ethylene tetrafluoride resin or a hydrophilic polyvinylidene fluoride resin is preferred from the viewpoint of, for example, strength and chemical resistance.

An excessively large pore diameter causes insufficient containment. An excessively small pore diameter causes difficulties in gas permeation, which requires high pressure for a sufficient permeation rate. Therefore, the porous material used for the oxygen separation membrane according to the present invention preferably has a membrane shape with a pore diameter (average pore diameter) of 0.1 to 0.5 µm.

Specifically, the pore diameter (µm) is 0.10, 0.20, 0.22, 0.30, 0.40, 0.45, or 0.50.

The thickness of the porous material is preferably as thin as possible to achieve high permeability, though the porous material needs to have a strength required for its use. To achieve high permeability, the upper limit of the thickness is preferably 200 µm or less and more preferably 50 µm or less.

Specifically, the upper limit of the thickness (µm) is 200, 180, 160, 150, 140, 130, 120, 110, 100, 90, 80, 70, 60, 50, 40, 30, 25, 20, 15, 10, or 5.

The porous material that can be used in the present invention is, for example, a commercially available membrane filter used in Examples.

### (Method for producing oxygen separation membrane)

The oxygen separation membrane according to the present invention is an impregnated membrane in which a liquid complex constituted by the metal salen complex such as a cobalt salen complex, the first ionic liquid, and the optionally added second ionic liquid is contained in the porous material. The oxygen separation membrane is produced through a step of preparing a mixed liquid containing a metal salen complex, a first ionic liquid, and a second ionic liquid that is optionally added and a step of causing the mixed liquid to be contained in a porous material.

In one example of the step of preparing a mixed liquid, a first ionic liquid is mixed with a metal salen complex in an atmosphere containing oxygen to dissolve the metal salen complex in the first ionic liquid. Then, a second ionic liquid is optionally added thereto and stirring is further performed to prepare a mixed liquid.

The metal salen complex and the first ionic liquid are desirably mixed with each other in an equimolar manner because the largest amount of oxygen can be coordinated when the metal salen complex and the first ionic liquid are coordinated at 1:1. The number of moles of the second ionic liquid is larger than that of the metal salen complex, and is desirably determined in consideration of the sufficient amount for appropriately controlling the viscosity for impregnation of the porous material in the production of the impregnated membrane. Thus, the ratio of the metal salen complex, the first ionic liquid, and the second ionic liquid in the mixed liquid is, for example, 1:1:3 on a molar basis.

In one example of the step of causing the mixed liquid to be contained in a porous material, the mixed liquid is thinly spread on a glass vessel such as a petri dish, and a porous material is placed on the glass vessel so that the entire membrane of the porous material is immersed. The porous material is immersed under a reduced pressure for one hour to remove air bubbles in the membrane and cause the mixed liquid to spread into the membrane. Then, the membrane is taken out of the mixed liquid, and a mixed liquid that adheres to the surface is removed to produce an impregnated membrane.

The impregnation of the porous material with the mixed liquid can be visually confirmed by a change in the color of the porous material after the impregnation. Furthermore, by checking the amounts of gas that permeates through the porous material before and after the impregnation, it can be confirmed whether the pores are sufficiently impregnated with the mixed liquid.

### (Physical properties of oxygen separation membrane)

The oxygen separation membrane according to the present invention preferably has an oxygen permeability of 10 Barrer (1 Barrer = 1 × 10⁻¹⁰ cm³(STP)·cm/(cm²·s·cmHg)) or more at an oxygen partial pressure in the air, and the ratio of a permeation separation factor of oxygen to a permeation separation factor of nitrogen is preferably 2 or more.

If the oxygen permeability is less than 10 Barrer, the movement of oxygen in the membrane is difficult, which sometimes does not sufficiently produce the advantageous effects of the invention. If the ratio of a permeation separation factor of oxygen to a permeation separation factor of nitrogen is less than 2, the separation efficiency of oxygen is decreased, which sometimes does not sufficiently produce the advantageous effects of the invention.

Specifically, the oxygen permeability (Barrer) is 10, 15, 20, 25, 30, 35, 40, 45, 50, 55, 60, 65, 70, 75, 80, 85, 90, 95, 100, 120, 140, 160, 180, 200, 300, 400, 500, 600, 700, 800, 900, or 1000.

Specifically, the ratio of a permeation separation factor of oxygen to a permeation separation factor of nitrogen is 2.0, 2.1, 2.2, 2.3, 2.4, 2.5, 2.6, 2.7, 2.8, 2.9, 3.0, 3.2, 3.4, 3.6, 3.8, 4.0, 4.2, 4.4, 4.6, 4.8, 5.0, 6.0, 7.0, 8.0, 9.0, 10.0, 11.0, 12.0, 13.0, 14.0, 15.0, 16.0, 17.0, 18.0, 19.0, or 20.0.

These physical properties can be determined by using, for example, a gas permeation tester configured to measure a gas permeation amount by a sweep method as described in Examples.

### EXAMPLES

Hereafter, the present invention will be specifically described based on Examples, but the present invention is not limited thereto.

### (Example 1)

### [Preparation of ionic liquid]

To 100 ml of ethanol, 5.64 g (10 mmol) of trihexyl(tetradecyl)phosphonium bromide (manufactured by Sigma-Aldrich, purity > 95%) and 20 g of an anion exchange resin (manufactured by Sigma-Aldrich, Amberlite (registered trademark) IBN78 hydroxide form) were added and stirred to cause a substitution reaction into hydroxides. Then, the reaction solution was separated by suction filtration, and an aqueous solution prepared by dissolving 0.98 g (11 mmol) of N-methylglycine (manufactured by Tokyo Chemical Industry Co., Ltd., purity > 98%) in 20 ml of pure water was added thereto to cause a reaction. By removing the solvent and an unreacted product through vacuum concentration, 4.88 g of an ionic liquid constituted by a trihexyl(tetradecyl)phosphonium cation and an N-methylglycine anion was prepared.

### [Preparation of mixed liquid]

Subsequently, 1.30 g (active component 2.3 mmol) of the prepared ionic liquid serving as a first ionic liquid and 2.35 g (7.2 mmol) of N,N'-bis(salicylidene)ethylenediaminocobalt(II) (manufactured by Tokyo Chemical Industry Co., Ltd., purity > 95%) were added to 50 ml of ethanol and stirred at room temperature for three hours. By removing the solvent and an unreacted product through vacuum concentration, 1.82 g of an intended liquid having a cobalt salen complex structure was prepared.

Herein, the coordination of an anion of the ionic liquid to a cobalt ion of the cobalt salen complex was confirmed by, for example, visible and ultraviolet spectrophotometry or observation of a coloration state.

To 0.91 g (active component 1.0 mmol) of the prepared liquid, 2.26 g (3.0 mmol) of trihexyl(tetradecyl)phosphonium·bis(trifluoromethanesulfonyl )imide serving as a second ionic liquid was added and stirred to prepare an intended mixed liquid in which the mixing molar ratio of the cobalt salen complex, the first ionic liquid, and the second ionic liquid was 1:1:3.

Note that the trihexyl(tetradecyl)phosphonium·bis(trifluoromethanesulfonyl )imide was synthesized with reference to the previous report (Dr. Tom Vander Hoogerstraete and two others, "Selective Single-Step Separation of a Mixture of three Metal Ions by a Triphasic Ionic-Liquid-Water-Ionic-Liquid Solvent Extraction System", Chemistry-A European Journal, 2015, Vol. 21, pp. 11757-11766).

### [Production of impregnated membrane]

The prepared mixed liquid was inserted into a petri dish, and a hydrophilic PTFE filter (manufactured by ADVANTEC, product name: H010A047A) having a thickness of 35 µm, a diameter of 47 mm, and a pore diameter of 0.10 µm was immersed in the mixed liquid. In this state, degassing treatment was performed under reduced pressure for about one hour to cause the mixed liquid to sufficiently infiltrate into pores of the membrane. The immersed membrane was taken out, and a mixed liquid that adhered to the surface was removed to produce an impregnated membrane (oxygen separation membrane).

### [Evaluation of oxygen permeable membrane]

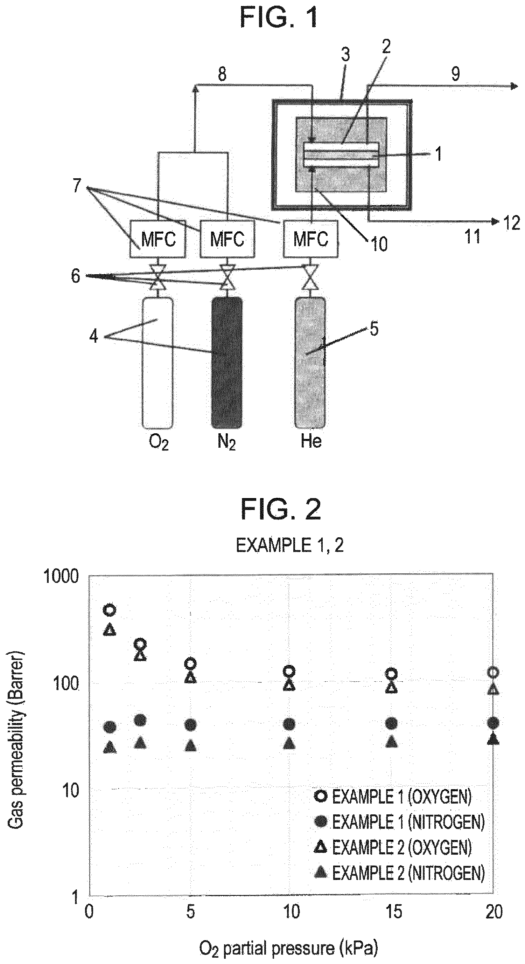

The performance of the oxygen separation membrane was evaluated using a gas permeation tester configured to measure a gas permeation amount by a sweep method illustrated in Fig. 1. In this method, a target gas is introduced into one side of a gas permeation test cell and an inert gas is introduced into the other side of the gas permeation test cell at an equal pressure, and the amount of a target gas that permeates through the membrane is measured using a detector.

Specifically, an oxygen separation membrane 1 was attached to a gas permeation test cell 2. In the thermostatic oven 3, an evaluation gas introduction line 8 and an evaluation gas release line 9 were connected to the upstream side of the gas permeation test cell 2, and an inert gas introduction line 10 and a permeated gas line 11 were connected to the downstream side of the gas permeation test cell 2. The temperature of the thermostatic oven 3 was controlled so that the evaluation temperature was set to 30°C.

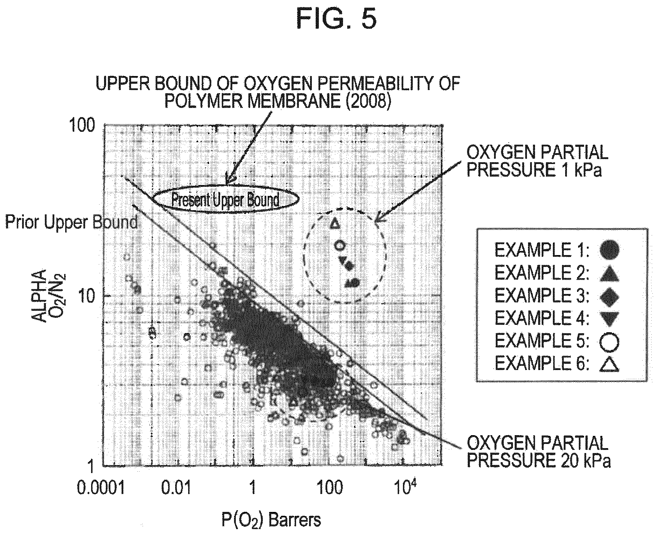

After the temperature was kept constant, evaluation gases 4 and an inert gas 5 were introduced through the regulators 6 under the following experimental conditions using mass flow controllers (MFC) 7. The permeated gas passed through the permeated gas line 11 and was detected by a micro-gas chromatograph (µGC, manufactured by Varian (current: Agilent), model: 490-GC) 12. The analysis and quantification of components were performed. Fig. 2 and Fig. 5 illustrate the obtained results.

Tables 1 and 2 illustrate the oxygen permeability, the nitrogen permeability, and the ratio of a permeation separation factor of oxygen to a permeation separation factor of nitrogen (oxygen/nitrogen selectivity) at oxygen partial pressures of 20 kPa and 1 kPa.

The oxygen partial pressure 20 kPa corresponds to a flow ratio of oxygen:nitrogen = 1:4, which is equivalent to the air composition. The evaluation result under this condition corresponds to the membrane performance for separation of oxygen from the air.

### (Experimental conditions)

Temperature: 303 K
Pressure Supply: 100 kPa
Sweep: 100 kPa
Gas flow rate Total: 100 ml/min

| | |
|---|---|
| Supply | O₂: 1 to 20 ml/min |
| | N₂: (balance) ml/min |
| Sweep | He: 40 ml/min |
| O₂ partial pressure: | 1 to 20 kPa |

### (Example 2)

An ionic liquid constituted by a trihexyl(tetradecyl)phosphonium cation and an N-methylglycine anion was prepared in the same manner as in Example 1.

Subsequently, 1.75 g (active component 3.1 mmol) of the prepared ionic liquid and 0.48 g (1.5 mmol) of N,N'-bis(salicylidene)ethylenediaminocobalt(II) (manufactured by Tokyo Chemical Industry Co., Ltd., purity > 95%) were added to 50 ml of ethanol and stirred at room temperature for three hours. Then, deoxidation treatment with nitrogen bubbling was performed for one hour. By removing the solvent and an unreacted product through vacuum concentration, 1.77 g of an intended liquid having a cobalt salen complex structure was prepared.

The production of an impregnated membrane and the evaluation of an oxygen permeable membrane were performed under the same conditions and through the same procedure as in Example 1.

Figs. 2 and 5 and Tables 1 and 2 illustrate the results of the membrane evaluation.

### (Example 3)

### [Preparation of ionic liquid]

To 100 ml of ethanol, 3.96 g (10 mmol) of tributyl-n-octylphosphonium bromide (manufactured by Tokyo Chemical Industry Co., Ltd., purity > 98%) and 20 g of an anion exchange resin (manufactured by Sigma-Aldrich, Amberlite (registered trademark) IBN78 hydroxide form) were added and stirred to cause a substitution reaction into hydroxides. Then, the reaction solution was separated by suction filtration, and an aqueous solution prepared by dissolving 0.98 g (11 mmol) of N-methylglycine (manufactured by Tokyo Chemical Industry Co., Ltd., purity > 98%) in 20 ml of pure water was added thereto to cause a reaction. By removing the solvent and an unreacted product through vacuum concentration, 3.40 g of an ionic liquid constituted by a tributyl-n-octylphosphonium cation and an N-methylglycine anion was prepared.

### [Preparation of mixed liquid]

Subsequently, 1.52 g (active component 3.8 mmol) of the prepared ionic liquid serving as a first ionic liquid and 3.63 g (11.2 mmol) of N,N'-bis(salicylidene)ethylenediaminocobalt(II) (manufactured by Tokyo Chemical Industry Co., Ltd., purity > 95%) were added to 50 ml of ethanol and stirred at room temperature for three hours. By removing the solvent and an unreacted product through vacuum concentration, 2.22 g of an intended liquid having a cobalt salen complex structure was prepared.

To 1.10 g (active component 1.5 mmol) of the prepared liquid, 2.71 g (4.5 mmol) of tributyl-n-octylphosphonium·bis(trifluoromethanesulfonyl)imide serving as a second ionic liquid was added and stirred to prepare an intended mixed liquid in which the mixing molar ratio of the cobalt salen complex, the first ionic liquid, and the second ionic liquid was 1:1:3.

Note that the tributyl-n-octylphosphonium·bis(trifluoromethanesulfonyl)imide was synthesized by the same method as that of the trihexyl(tetradecyl)phosphonium·bis(trifluoromethanesulfonyl )imide in Example 1.

The production of an impregnated membrane and the evaluation of an oxygen permeable membrane were performed under the same conditions and through the same procedure as in Example 1.

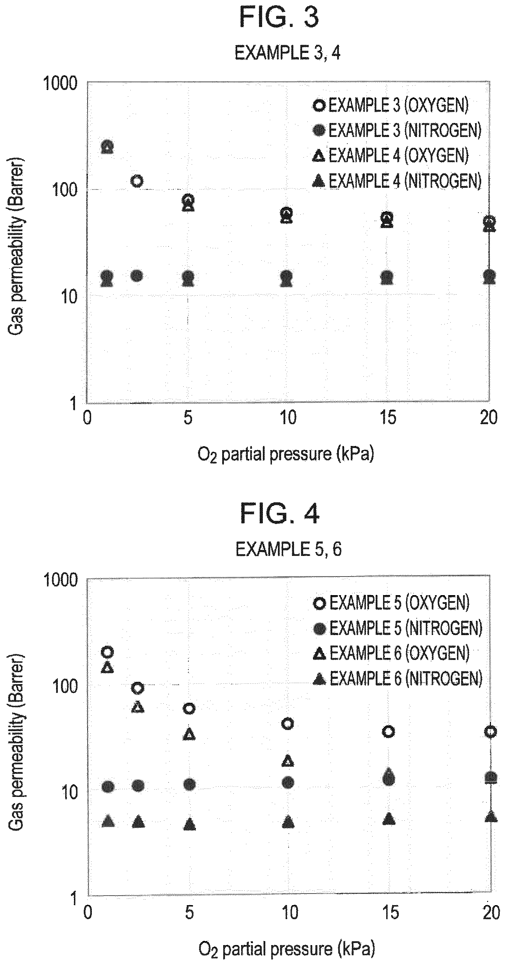

Figs. 3 and 5 and Tables 1 and 2 illustrate the results of the membrane evaluation.

### (Example 4)

An ionic liquid constituted by a tributyl-n-octylphosphonium cation and an N-methylglycine anion was prepared in the same manner as in Example 3.

Subsequently, 1.60 g (active component 4.0 mmol) of the prepared ionic liquid and 0.66 g (2.0 mmol) of N,N'-bis(salicylidene)ethylenediaminocobalt(II) (manufactured by Tokyo Chemical Industry Co., Ltd., purity > 95%) were added to 50 ml of ethanol and stirred at room temperature for three hours. Then, deoxidation treatment with nitrogen bubbling was performed for one hour. By removing the solvent and an unreacted product through vacuum concentration, 1.85 g of an intended liquid having a cobalt salen complex structure was prepared.

The production of an impregnated membrane and the evaluation of an oxygen permeable membrane were performed under the same conditions and through the same procedure as in Example 1.

Figs. 3 and 5 and Tables 1 and 2 illustrate the results of the membrane evaluation.

### (Example 5)

### [Synthesis of phosphonium cation salt]

To a three-necked flask, 100 g of a 1.0 M triethylphosphine solution (manufactured by Sigma-Aldrich) was added. The solution was heated for reflux. In this reflux state, 15.58 g of 1-pentyl bromide (manufactured by Tokyo Chemical Industry Co., Ltd., purity 98%) was added dropwise thereto and stirred at 80°C for about six hours to cause a reaction. After the completion of the reaction, the generation of a white solid was observed.

After the obtained reaction solution was left to cool at room temperature, the reaction solution was added dropwise to about 300 ml of hexane (manufactured by Wako Pure Chemical Industries, Ltd., purity 96%) under stirring and then stirred for about one hour to sufficiently precipitate a solid. The resulting suspension was left to stand overnight to settle the solid. Then, the solid product was transferred to a recovery flask, and hexane was completely removed using an evaporator under reduced pressure at 40°C for about three hours to obtain 16.72 g of a white solid of triethylpentylphosphonium bromide.

### [Preparation of ionic liquid]

To 100 ml of ethanol, 2.70 g (10 mmol) of the synthesized triethylpentylphosphonium bromide and 20 g of an anion exchange resin (manufactured by Sigma-Aldrich, Amberlite (registered trademark) IBN78 hydroxide form) were added and stirred to cause a substitution reaction into hydroxides. Then, the reaction solution was separated by suction filtration, and an aqueous solution prepared by dissolving 0.98 g (11 mmol) of N-methylglycine (manufactured by Tokyo Chemical Industry Co., Ltd., purity > 98%) in 20 ml of pure water was added thereto to cause a reaction. By removing the solvent and an unreacted product through vacuum concentration, 2.27 g of an ionic liquid constituted by a triethylpentylphosphonium cation and an N-methylglycine anion was prepared.

### [Preparation of mixed liquid]

Subsequently, 1.40 g (active component 5.0 mmol) of the prepared ionic liquid serving as a first ionic liquid and 4.88 g (15 mmol) of N,N'-bis(salicylidene)ethylenediaminocobalt(II) (manufactured by Tokyo Chemical Industry Co., Ltd., purity > 95%) were added to 50 ml of ethanol and stirred at room temperature for three hours. By removing the solvent and an unreacted product through vacuum concentration, 2.44 g of an intended liquid having a cobalt salen complex structure was prepared.

To 1.22 g (active component 2.0 mmol) of the prepared liquid, 2.81 g (6.0 mmol) of triethylpentylphosphonium·bis(trifluoromethanesulfonyl)imide serving as a second ionic liquid was added and stirred to prepare an intended mixed liquid in which the mixing molar ratio of the cobalt salen complex, the first ionic liquid, and the second ionic liquid was 1:1:3.

Note that the triethylpentylphosphonium·bis(trifluoromethanesulfonyl)imide was synthesized by the same method as that of the trihexyl(tetradecyl)phosphonium·bis(trifluoromethanesulfonyl )imide in Example 1.

The production of an impregnated membrane and the evaluation of an oxygen permeable membrane were performed under the same conditions and through the same procedure as in Example 1.

Figs. 4 and 5 and Tables 1 and 2 illustrate the results of the membrane evaluation.

### (Example 6)

An ionic liquid constituted by a triethylpentylphosphonium cation and an N-methylglycine anion was prepared in the same manner as in Example 5.

Subsequently, 1.38 g (active component 5.0 mmol) of the prepared ionic liquid and 0.81 g (2.5 mmol) of N,N'-bis(salicylidene)ethylenediaminocobalt(II) (manufactured by Tokyo Chemical Industry Co., Ltd., purity > 95%) were added to 50 ml of ethanol and stirred at room temperature for three hours. Then, deoxidation treatment with nitrogen bubbling was performed for one hour. By removing the solvent and an unreacted product through vacuum concentration, 1.76 g of an intended liquid having a cobalt salen complex structure was prepared.

The production of an impregnated membrane and the evaluation of an oxygen permeable membrane were performed under the same conditions and through the same procedure as in Example 1.

Figs. 4 and 5 and Tables 1 and 2 illustrate the results of the membrane evaluation.

**[Table 1]**

| Oxygen partial pressure 20 kPa | Oxygen permeability (Barrer) | Nitrogen permeability (Barrer) | Oxygen/nitrogen selectivity |
|---|---|---|---|
| Example 1 | 118.7 | 39.5 | 3.0 |
| Example 2 | 83.8 | 28.5 | 2.9 |
| Example 3 | 48.6 | 15.1 | 3.2 |
| Example 4 | 44.8 | 14.0 | 3.2 |
| Example 5 | 33.3 | 12.0 | 2.8 |
| Example 6 | 12.1 | 5.2 | 2.3 |

**[Table 2]**

| Oxygen partial pressure 1 kPa | Oxygen permeability (Barrer) | Nitrogen permeability (Barrer) | Oxygen/nitrogen selectivity |
|---|---|---|---|
| Example 1 | 478.7 | 38.5 | 12.4 |
| Example 2 | 318.2 | 25.0 | 12.7 |
| Example 3 | 253.8 | 15.4 | 16.5 |
| Example 4 | 245.3 | 13.9 | 17.6 |
| Example 5 | 204.3 | 10.7 | 19.1 |
| Example 6 | 147.5 | 5.2 | 28.6 |

As a result of the comparison between these results and polymer materials in the previously reported paper (refer to Fig. 1 on p. 392 in NPL 1) that summarizes the relationship between the permeability of oxygen separation membranes and the permeation selectivity of oxygen and nitrogen, it can be confirmed that the oxygen separation membrane according to the present invention exhibits the performance equivalent to that of known membranes in terms of air composition, and has a permeability and a selective permeability higher than those of known membranes when the oxygen partial pressure is low.

### Industrial Applicability

By modularizing the oxygen separation membrane according to the present invention, high-concentration oxygen can be obtained with low energy. The oxygen separation membrane can be used in a wide range of fields that require air and oxygen, such as combustion systems, health care concerned with respiration, and air-conditioning systems.

### Reference Signs List

- 1: oxygen separation membrane
- 2: gas permeation test cell
- 3: thermostat oven
- 4: evaluation gas
- 5: inert gas
- 6: regulator
- 7: mass flow controller (MFC)
- 8: evaluation gas introduction line
- 9: evaluation gas release line
- 10: inert gas introduction line
- 11: permeated gas line
- 12: micro-gas chromatograph

## Claims

1. An oxygen separation membrane comprising a porous material and a liquid complex contained in the porous material,
wherein the complex contains a metal salen complex or a derivative thereof and a first ionic liquid,
the first ionic liquid is constituted by an anion having an amine structure and an imidazolium cation, an aliphatic quaternary phosphonium cation, or an ammonium cation that have alkyl chains, alkylene oxide chains, or an alkyl ether chains with each 2 to 20 carbon atoms, and
the anion of the first ionic liquid is axially coordinated to a central metal ion of the metal salen complex or the derivative thereof.

2. The oxygen separation membrane according to Claim 1, wherein the first ionic liquid contains a secondary amine anion.

3. The oxygen separation membrane according to Claim 2, wherein the first ionic liquid contains an N-methylglycine anion.

4. The oxygen separation membrane according to any one of Claims 1 to 3, wherein the complex further contains a second ionic liquid having compatibility with the first ionic liquid, and the anion of the first ionic liquid is coordinated as one of axial ligands of the central metal ion of the metal salen complex or the derivative thereof.

5. The oxygen separation membrane according to Claim 4, wherein the second ionic liquid contains a bis(trifluoromethanesulfonyl)imide anion.

6. The oxygen separation membrane according to any one of Claims 1 to 5, wherein the first ionic liquid and/or the second ionic liquid contains any one of a trihexyl(tetradecyl)phosphonium cation, a tributyl-n-octylphosphonium cation, and a triethylpentylphosphonium cation.

7. The oxygen separation membrane according to any one of Claims 1 to 6, wherein the metal salen complex is N,N'-bis(salicylidene)ethylenediaminocobalt(II).

8. The oxygen separation membrane according to any one of Claims 1 to 7, wherein the porous material is a hydrophilic ethylene tetrafluoride resin or a hydrophilic polyvinylidene fluoride resin.

9. The oxygen separation membrane according to any one of Claims 1 to 8, wherein the porous material has a membrane shape with a pore diameter of 0.1 to 0.5 µm.

10. The oxygen separation membrane according to any one of Claims 1 to 9, wherein the oxygen separation membrane has an oxygen permeability of 10 Barrer (1 Barrer = 1 × 10⁻¹⁰ cm³(STP)·cm/(cm²·s·cmHg)) or more at an oxygen partial pressure in air, and a ratio of a permeation separation factor of oxygen to a permeation separation factor of nitrogen is 2 or more.
